# EUROPEAN PATENT SPECIFICATION

(11) **EP 4 547 805 B1**
(45) Date of publication and mention of the grant of the patent: **15.10.2025**
(21) Application number: 23731278.0
(22) Date of filing: 07.06.2023
(51) Int. Cl.: C11D 1/94, C11D 3/22, A61K 8/73, A61K 8/46, A61K 8/42, A61K 8/36, A61K 8/34, A61K 8/365

(54) **STABLE WASH COMPOSITION WITH BIODEGRADABLE THICKENER**
STABILES WASCHMITTEL MIT BIOLOGISCH ABBAUBAREM VERDICKUNGSMITTEL
COMPOSITION DE LAVAGE STABLE AVEC ÉPAISSISSANT BIODÉGRADABLE

(30) Priority: 30.06.2022 US 202263357321 P; 06.09.2022 EP 22194216
(43) Date of publication of application: 07.05.2025
(73) Proprietor: Unilever IP Holdings B.V., 6708 WH Wageningen (NL); Unilever Global IP Limited, Wirral, Merseyside CH62 4ZD (GB)
(72) Inventor: YUAN, Mingjun, 6708 WH Wageningen (NL); VASUDEVAN, Tirucherai Varahan, 6708 WH Wageningen (NL)
(74) Representative: Askew, Sarah Elizabeth
(86) International application number: PCT/EP2023/065242
(87) International publication number: WO 2024/002646

(56) References cited:
- WO-A1-2022/101097
- CN-A- 108 042 480
- US-A1- 2018 311 135
- US-A1- 2021 290 517
- US-A1- 2022 062 118

## Description

### Field of the Invention

The present invention is directed to a stable wash composition comprising biodegradable thickeners. More particularly, the invention is directed to a wash composition comprising water, surfactant and biodegradable thickeners comprising starch and cellulose. Such a composition surprisingly yields excellent lather and viscosity characteristics, rinses easily for a clean and filmless sensory feel, and is free of syneresis, discoloration and malodor after being stored at elevated temperatures, even when formulated with low surfactant amounts and substantially free of soaps, sulfates, isethionates and/or synthetic thickeners like ammonium acryloyldimethyltaurate vinylpyrrolidone copolymer, alkyl acrylate cross polymers or the like.

### Background of the Invention

Wash compositions are typically employed to cleanse surfaces, like skin, and often to reduce shine associated with sebum produced in specialized epithelial cells known as sebocytes. They are also used to minimize bacteria on surfaces, like the hands and face, whereby washing is viewed as the most effective way to prevent the spread of germs and bacteria. In fact, experts believe that periodic washing throughout the day can reduce the number of consumers catching colds by about 50%.

Consumers know it is generally good practice to regularly clean their hands and not touch their face, eyes and mouth in order to minimize the risk of getting sick. Information provided by the Centers for Disease Control and Prevention suggests that the COVID-19 (coronavirus) pandemic is a direct result of a virus that is more efficient and severe than influenza and that spreads rapidly from person to person via respiratory droplets. During such a pandemic, consumers understand and medical professionals emphasize it is in everyone's best interest to consistently wash their hands and face, and inanimate surfaces they come in contact with prior to use.

With consumers washing more often, it is highly desirable to develop wash compositions that are not only good for the consumer but enjoyable to use and friendlier towards the environment. This invention, therefore, is directed to a stable wash composition comprising water, surfactant and biodegradable thickener comprising starch and cellulose. The wash composition surprisingly yields excellent lather and viscosity characteristics, rinses easily for a clean and filmless sensory feel, and is free of syneresis, discoloration and malodor after being stored at elevated temperatures, even when formulated with low surfactant amounts and substantially free of soaps, sulfates, isethionates and synthetic thickeners like ammonium acryloyldimethyltaurate vinylpyrrolidone copolymer, alkyl acrylate cross polymers or the like.

### Additional Information

Efforts have been disclosed for making wash compositions. In U.S. Patent Application No. 2019/008738A1, personal care compositions with cationic surfactant, nonionic surfactant and a polyacrylate cross polymer structuring agent are described. The wash compositions can include carrageenan as a thickener.

Other efforts have been described for making wash compositions. In U.S. Patent Application No. 2019/0359735A1, water soluble hybrid polymers suitable for use in a body wash are described.

Still other efforts have been described for making wash compositions. In U.S. Patent No. 9,549,885, scalp care compositions that can include synthetic rheology modifiers like acrylamide/ammonium acrylates/C10-C30 alkyl acrylate crosslinked polymer or acrylates/steareth-20 itaconate copolymer are described.

Further wash compositions comprising starch and cellulose compounds are disclosed in US 2021/290517 A1.

None of the additional information describes a composition with biodegradable thickener comprising starch and cellulose as claimed herein.

### Summary of the Invention

The present invention is directed to a wash composition comprising:
a) anionic surfactant;
b) amphoteric surfactant, zwitterionic surfactant or both;
c) 0 to 4% by weight nonionic surfactant; and
d) 1.7 to 8.5% by weight thickeners, the thickener having starch and cellulose, the starch being a hydroxypropyl starch, distarch phosphate or mixture thereof and the cellulose being a microcrystalline cellulose selected from the group consisting of carboxymethylcellulose, hydroxymethylcellulose, hydroxypropylcellulose, hydroxypropylmethylcellulose, hydroxyethylcellulose or a mixture thereof,
wherein when the thickeners are present at 1.7 to less than or equal to 4.5% by weight of the composition, the weight ratio of starch:cellulose is greater than 0 and less than 4.5, and further wherein when the thickener is present at greater than 4.5% to 8.5% by weight, the weight ratio of starch:cellulose is > 0 and less than 15, and:
i) the composition has from 3.0 to 15%, and preferably, from 4 to 13%, and most preferably, 4.5 to 11% by weight total surfactant;
ii) the total surfactant comprises from 8 to 70%, and preferably, 10 to 65%, and most preferably, 15 to 60% by weight amphoteric surfactant, zwitterionic surfactant or both based on total weight of anionic, and zwitterionic and/or amphoteric surfactant in the composition; and
iii) the composition has a pH from greater than 4.25 to 8, and preferably, from 4.5 to 6.5, and most preferably, from 4.75 to 6,
the composition is substantially free of sulfate and isethionate, wherein substantially free means each individual component making up less than 0.2% by weight of the total composition.

Herein is also described the use of a mixture of starch and cellulose to thicken and stabilize a wash composition.

Herein is also described a method of using a composition having a mixture of starch and cellulose according to the first three aspects of the invention to wash a surface.

All other aspects of the present invention will more readily become apparent from the description and examples which follow.

Skin, as used herein, is meant to include skin on the arms (including underarms), face, feet, neck, chest, hands, legs, buttocks and scalp (including hair). Cellulose includes a water insoluble polysaccharide composed of chains of glucose molecules linked via *beta* 1-4 glycosidic bonds or linkages. Cellulose also includes polysaccharide that is water soluble when chemically or physically modified and water dispersible when used with cellulose that is water soluble. Starch, as used herein, means a water-soluble polysaccharide made up of glucose molecules that are joined by linkages that include *alpha* 1-4 and/or 1-6 glycosidic bonds or linkages. Wash composition means a composition suitable for use on a surface, including a hard surface such as a table, countertop, appliance, window, ceramic fixture (like a toilet or sink), automobile and floor. Surface also includes skin, and preferably, is skin.

The wash composition of the present invention, therefore, is suitable to be a home care composition, shampoo, make-up remover, facial wash or personal care and liquid body or hand wash. Preferably, the wash composition of the present invention is a body wash or hand wash that is ready for topical application and to be wiped or washed off, and preferably, washed off with water. The wash composition may, optionally, comprise medicinal or therapeutic agents, but preferably, is a wash which is a cleaning and/or cosmetic wash that is a non-therapeutic wash to wash of, for example, dirt, oils, and/or bacteria that can cause staining and/or malodour. As hereinafter described, the wash composition of the present invention may optionally comprise skin benefit ingredients added thereto such as vitamins and/or derivatives thereof, resorcinols, retinoic acid precursors, colorants, moisturizers, sunscreens, mixtures thereof or the like. The skin benefit ingredients (or agents) may be water or oil soluble. If used, oil soluble skin benefit agents typically make up to 1.5% by weight of the wash composition whereby water-soluble skin benefit agents, when used, can make up to 10% by weight of the wash composition. The wash composition has a pH from greater than 4.25 to 8, and preferably, 4.5 to 6.5, and most preferably, 4.75 to 6.0. Viscosity, unless noted otherwise, is taken with a Discovery HR-2 Rheometer using sand blasted plates having a 1000 micron gap and a first shear rate S_{A} of 4 s⁻¹ for a first viscosity V_{A} and a second shear rate S_{B} of 10 s⁻¹ for a second viscosity V_{B}, both at 25°C and 20 second intervals. Viscosity is reported in millipascal seconds (1 millipascal seconds = 1 centipoise (cps)). Stable, as used herein, means no phase separation, discoloration and malodour generation from the wash composition after being stored for at least two (2) weeks at 50 °C, and preferably, at least one (1) month at 45°C, and more preferably, from 1 to 4 months at 45 °C. Rinses easily for a clean and filmless sensory feel means washes off easily without leaving a sticky or tacky residue feeling as determined by trained panelists. Biodegradable, as used herein, means the breakdown of organic matter like starch and cellulose by microorganisms, such as bacteria and fungi. The term comprising is meant to encompass the terms consisting essentially of and consisting of. For the avoidance of doubt, and for illustration, the composition of this invention comprising surfactant and biodegradable thickener is meant to include a composition consisting essentially of the same and a composition consisting of the same. Low surfactant level means no more than 15% by weight, and preferably, no more than 13% by weight, and most preferably, no more than 11% (or 8.5%) by weight based on total weight of the composition. Except in the operating comparative examples, or where otherwise explicitly indicated, all numbers in this description indicating amounts or ratios of materials or conditions and/or physical properties of materials and/or use are to be understood as modified by the word "about". All ranges defined herein are meant to include all ranges subsumed therein unless otherwise stated.

### Detailed Description of the Invention

As to the biodegradable thickener suitable for use, the same comprises starch and cellulose where the thickener is not required to be synthetically manufactured and can completely decompose or disintegrate in the environment. In an embodiment of the invention, at least 80% by weight of the biodegradable thickener is starch and cellulose. In another embodiment, at least 85% by weight of the biodegradable thickener is starch and cellulose. In even another embodiment, at least 90 to 100% by weight of the biodegradable thickener is starch and cellulose. In still another embodiment, from 90 to 97% or 92 to 96% or 100% by weight of the biodegradable thickener is starch and cellulose. In still another embodiment, the thickener used in the wash composition is 80 to 100%, and preferably, 90 to 100%, and most preferably, 95 to 100% (or 100%) by weight biodegradable thickener.

The wash composition of the present invention will comprise from 1.7 to 8.5% by weight of the thickener, and preferably, from 3.5 to 7.5%, and most preferably, from 3.75 to 6.0% by weight of the thickener. In another embodiment of the invention, cellulose makes from 12.5 to 65% or from 13 to 50% or from 15 to 35% by weight of the total weight of starch and cellulose in the thickener. In an embodiment of the invention, 100% of the biodegradable thickener is starch and cellulose where cellulose makes up from 15 to 35% by weight of the total weight of the thickener.

In a further embodiment, 100% by weight of the biodegradable thickener is starch and cellulose whereby the same makes up from 4.75 to 6.5%, and preferably, from 5.0 to 6.0%, and most preferably, from 5 to 5.5% by weight of the wash composition. In yet another embodiment, starch and cellulose (i.e., starch:cellulose) are at a weight ratio from 1.5 to 12, and preferably, from 2 to 10, and most preferably, from 2.25 to 9.25 or 4 to 8.5 or 5.5 to 7.5, depending on the amount of the thickener as indicated in claim 1.

Salt or electrolyte, like Na, Mg, Ca and/or ammonium chloride make(s) up from 0 to 2.5%, and preferably, from 0.1 to 1.35%, and most preferably, from 0.2 to 1% by weight of the wash composition. In another embodiment, the wash composition comprises less than 0.5% or less than 0.25% by weight or no (0.0% by weight) salt or electrolyte.

As to the biodegradable thickeners suitable for use, the starches include those that may be chemically or physically modified by one or more art recognized techniques like oxidations, crosslinking reactions, gelatinizations, esterification, etherification, amidation and art recognized treatments that include temperature variations. Distarch phosphates or compounds rich in distarch phosphate are starches often included for use. Starch hydrolysates are also suitable for use. In an embodiment of the invention, the thickener used in the wash composition of the present invention does not comprise starch hydrolysate.

Such starches may originate or be derived from any plant source like corn, pea, potato, oat, rice, tapioca, sorghum, barley or wheat.

Starches suitable for use include starch and distarch phosphates like acetylated distarch phosphate, acetylated distarch adipate, starch sodium octenyl succinate, hydroxypropyl starch phosphate, hydroxypropyl distarch phosphate mixtures thereof or the like. Illustrative examples of starches suitable for use either alone or in mixtures include: StarDesign^{™} Care, StarDesign^{™} Care AV, StarDesign^{™} 05340, C☆EmTex^{®} 06328, PolarTex^{®}, C☆PolarTex^{®}, C☆HiForm^{™}, C☆HiForm A^{™}, HiForm Starch^{™}, StarDesign^{™}, Pure-Gel^{®} B980, Pure-Gel^{®} B990, Pure-Gel^{®} B992, Pure-Gel^{®} B994, FARMAL^{®} AF 1100, FARMAL^{®} CS 3757, FARMAL MS 6822, FARMAL^{™} GMS 2143, FARMAL^{®} MS 6892, FARMAL^{®} MS 6135, FARMAL^{®} GMS 2141, FARMAL^{®} CS 21A, FARMAL^{®} CS 21R, FARMAL^{®} MS 6135, FARMAL^{®} GS 1653, FARMAL^{®} CS 3757, FARMAL^{®} GMS 2141, FARMAL^{®} CS 3001, FARMAL CS 3410, FARMAL^{®} CS 3400, FARMAL^{®} CS 3650, FARMAL^{®} WS 4400, FARMAL^{®} GMS 2141, Nativacare^{™} 8600, Nativacare^{™} 5600, Nativacare^{™} 9360, Nativacare^{™} 9230, Nativacare^{™} 9330; AGENAFLO 9050, AGENAFLO OS 9051, AGENAFLO TS, AGENAJEL 20.313, AGENAJEL 20.306, AGENAJEL 20.350, AGENAMALT 20.222, AGENANOVA 30.326, DEXTRIN 20.901, AGENABON 20.219, STRUCTURE^{®} XL, STRUCTURE^{®} 2143, STRUCTURE^{®} 6892, STRUCTURE^{®} STYLE or the like. Hydroxypropyl distarch phosphate, sold as E1440 hydroxypropyl starch phosophate by Sinofi, is also suitable for use.

The preferred hydroxypropyl starch and distarch phosphates suitable for use include those made available from Grain Processing Corporation, Pure-Gel^{®} (e.g., B980, B990 food starch modified grade), StarDesign^{™} (e.g., C*xEmTex^{®}), from Cargill Corporation, Agenaflo (9050, 9051) by Agrana, Farmal^{®} (e.g., CS 3757) from Ingredion and (e.g., Zea Mays ST 005) by Roquette.

Other preferred starch phosphates, and in particular hydroxypropyl starch phosphates, or compounds rich in starch phosphate suitable for use are sold by Avebe under the Prejel^{™} name. These include VA-70-T AGGL (gelatinized hydroxypropyl cassava distarch phosphate), TK1 (gelatinized cassava distarch phosphate) and 200 (gelatinized acetyl cassava distarch phosphate).

As to the microcrystalline celluloses suitable for use as biodegradable thickeners, these include components as a carboxymethylcellulose, hydroxymethylcellulose, hydroxypropylcellulose, hydroxypropylmethyl cellulose, hydroxyethylcellulose or mixtures thereof.

Illustrative examples of the microcrystalline celluloses that may be used include Vivapur^{®} COS5, Vivapur^{®} COS6, Vivapur^{®} COS8, Vivapur^{®} CS 032 XV, Vivapur^{®} CS 152 HV, Vivapur^{®} CS 302 SV, Vivapur^{®} 12, Vivapur^{®} 14, Vivapur^{®} 101, Vivapur^{®} 102, Vivapur^{®} 301, Vivapur^{®} 200, Emcocel^{®} 50M, Emcocel^{®} 90M, Emcocel^{®} 90HD, Emcocel^{®} 200 LP made available from JRS Pharma GmbH & Co.

Others suitable for use include Avicel^{®} GP1412; Avicel^{®} 101, Avicel^{®} 102, Avicel^{®} 103, Avicel^{®} 112, Avicel^{®} 113, Avicel^{®} 301, Avicel^{®} 200, Avicel^{®} SMCC 50, Avicel^{®} SMCC 90, Avicel^{®} SMCC HD90, Avicel^{®} CL, Avicel^{®} DG, Avicel^{®} CE, Avicel^{®} HFE, Lattice^{®} NT-20, Lattice^{®} NTC-61, Lattice^{®} NT-50, Lattice^{®} NT-80, Lattice^{®} NT-100, Lattice^{®} NT-200, GRINDSTED^{®} Cellulose gum BAK, GRINDSTED^{®} Cellulose gum BEV, GRINDSTED^{®} Cellulose gum MAS, GRINDSTED^{®} Cellulose gum AMD, GRINDSTED^{®} Cellulose gum NMD, GRINDSTED^{®} Cellulose gum FZD, Methocel^{™} 240, Methocel^{™} E5, Methocel^{™} F4M, Methocel^{™} K4M, Methocel^{™} K15M, Methocel^{™} K15LV, Methocel^{™} F50, Methocel^{™} K100M, Methocel^{™} K 200M, Methocel^{™} 40-0100, Methocel^{™} 40-0202 and Methocel^{™} F50 and available from Dupont.

Comprecel^{®} 101, Comprecel^{®} 102, Comprecel^{®} 301, Comprecel^{®} 302 available from Ming Thai Chemical Co.; Microcel^{®} 101, Microcel^{®} 102, Microcel^{®} 200 available from Blanver, Farmoquimica; Farmal^{®} CMC 2700 F available from Ingredion; Arbalon^{®} R49, Arbalon^{®} R50 available from Lubrizol; Natrava^{®} Citrus Fibers, Arbalon^{®} Cellulose Liquid available from CP Kelco are also suitable for use. Even other celluloses that can be used include Exilva^{®} FM 02-V available from Borregaard; CelluForce NCC^{®} available from CelluForce; CELOVA^{®}; FiberDesign^{™} Sensation available from Cargill, Natrava^{®} Citrus Fiber available from by CPKelco; CELLOSIZE^{™} QP-10000H EUR, CELLOSIZE^{™} QP-100MH, CELLOSIZE^{™} QP-300, CELLOSIZE^{™} QP-4400H, EUR CELLOSIZE^{™} QP-15000H, CELLOSIZE^{™} QP-30000H, CELLOSIZE^{™} QP-52000H, CELLOSIZE^{™} EP-09 made available by Dow Chemical; Methyl Cellulose PMK-40YS and PMK-60YS available by First Continental International Inc; Carboxymethyl Cellulose (CMC), Poly Anionic Cellulose (PAC), Hydroxyethyl Cellulose (HEC), Hydroxypropyl Methylcellulose (HPMC), and Methyl Hydroxyethyl Cellulose (MHEC/HEMC) made available by Kingsun Chemicals Inc.; Hydroxypropyl MethylCellulose (HPMC), Methyl Cellulose (MC), Sodium CarboxymethylCellulose (CMC), High Substitute Hydroxypropyl Cellulose (H-HPC), Low Substitute Hydroxypropyl Cellulose (L-HPC), Ethyl Cellulose(EC), Hydroxyethyl Cellulose (HEC), Microcrystalline Cellulose (MCC), Polyanionic Cellulose (PAC), Hydroxypropyl Methyl Cellulose Phthalate (HPMC-P)/(Hypromellose phthalate), Hydroxypropyl Methyl Cellulose Acetate Succinate (HPMCAS), Croscarmellose Sodium, Powdered Cellulose made commercially available from SIDLEY CHEMICAL CO., LTD.

The preferred microcrystalline celluloses used in the present invention are those made commercially available from Dupont and JRS Pharma GmbH & Co.

The biodegradable thickeners preferred typically have a bulk density from 0.18 to 0.90, and preferably, from 0.2 to 0.8, and most preferably, from 0.22 to 0.70 g/ml (or 1000kg/m³⁾ where bulk density is the ratio of the mass of an untapped powder sample and its volume including the contribution of the interparticulate void volume (i.e., dependent on both the density of powder particles and the spatial arrangement of particles in a powder bed). Such thickeners also have a cloud point from 60 to 70°C and a weight average molecular weight from 75,000 to 1.5 million Daltons, and preferably, from 80,000 to 1.25 million Daltons, and most preferably, from 100,000 to 1.0 million Daltons.

Regarding the surfactants that may be used, the same are limited only to the extent that they are suitable for use in compositions that are topically applied to a surface, preferably skin.

Anionic surfactants suitable for use in the wash composition of the present invention include aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate.

The anionic may also include alkyl sulfosuccinates (including mono- and dialkyl, e.g., C₈-C₂₂ sulfosuccinates); acyl taurates (often methyl taurates), acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, alkyl glucosides and the like.

Sulfosuccinates may be monoalkyl sulfosuccinates having the formula:

R¹O₂CCH₂CH(SO₃M)CO₂M;

and amide-MEA sulfosuccinates of the formula:

R¹CONHCH₂CH₂O₂CCH₂CH(SO₃M)CO₂M

wherein R¹ ranges from C₈-C₂₂ alkyl.

Sarcosinates are generally indicated by the formula:

R²CON(CH₃)CH₂CO₂M,

wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described. The isethionates include C₈-C₁₈ acyl isethionates (including those which have a substituted head group such as a C₁₋₄ alkyl substitution, preferably methyl substitution). These esters are prepared by a reaction between alkali metal isethionate with mixed aliphatic fatty acids having from 6 to 18 carbon atoms and an iodine value of less than 20. Often at least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995.

This compound has the general formula:

R⁵C-O(O)-C(X)H-C(Y)H-(OCH₂-CH₂)ₘ-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

In an embodiment of the invention, an anionic surfactant suitable for use is sodium methyl lauroyl taurate, sodium methyl cocoyl taurate or a mixture thereof. Such anionic surfactants are commercially available from suppliers like Galaxy Surfactants, Clariant, Sino Lion and Innospec. Sodium methyl lauroyl taurate, is often the preferred anionic used.

The wash compositions can optionally contain glutamates and glycinates but as is the case with isethionates, they are substantially free of the same. If optionally used, the glycinates suitable include sodium lauroyl glycinate, sodium cocoyl glycinate, and glutamates suitable for use include sodium lauroyl glutamate, sodium cocoyl glutamate, sodium myristol glutamate, isopropyl lauroyl glutamate or mixtures thereof.

Amphoteric surfactants suitable for use in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate, sodium lauroamphoacetate, sodium cocoamphoacetate or mixtures thereof.

As to the zwitterionic surfactants that may be employed in the wash compositions of the present invention, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms generally comply with an overall structural formula: R⁶-[-C(O)-NH(CH₂)_{q}-]ᵣ-N-(R⁷-)(R⁸)A-B where R⁶ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants that may be used in the present invention and within the above general formula include simple betaines of formula:

R⁶-N⁺-(R⁷)(R⁸)CH₂CO₂⁻

and amido betaines of formula:

R⁶-CONH(CH₂)ₜ-N⁺-(R⁷)(R⁸)CH₂CO₂⁻

where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

A further possibility is that the zwitterionic surfactant is a sulphobetaine of formula:

R⁶--N⁺--(R ⁷) (R ⁸) (CH₂)₃SO₃⁻

or

R⁸-CONH(CH₂)ᵤ -N⁺-(R⁷)(R⁸)(CH₂)₃SO₃⁻

where u is 2 or 3, or variants of these in which --(CH₂)₃SO₃⁻ is replaced by-CH₂C(OH)(H)CH₂SO₃⁻.

In these formulae, R⁶, R⁷ and R⁸ are as previously defined.

Illustrative examples of the zwitterionic surfactants suitable for use include betaines like cocodimethyl carboxymethyl betaine, cocamidopropyl betaine and laurylamidopropyl betaine. An additional zwitterionic surfactant suitable for use includes cocamidopropyl hydroxy sultaine. Such surfactants are made commercially available from suppliers like Stepan Company, and it is within the scope of the invention to employ mixtures of the aforementioned surfactants. In a preferred embodiment, the zwitterionic surfactant used in the wash composition of this invention is cocamidopropyl betaine.

Nonionic surfactants may be used in the wash composition of the present invention. When used, nonionic surfactants are used at levels as low as 0.01, 0.5, 1, 1.5, 2, 3 or 4% by weight of the wash composition. The nonionics which may be used include, in particular, the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like. Cocamide monoethanolamine (MEA) is an often preferred nonionic surfactant suitable for use at levels from 0.2 to 0.65% by weight of the wash composition.

In an embodiment of the invention, nonionic surfactants optionally used can include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH-(CH₂)ₖ(CH₂CH₂O)_{z} H, where i, k are each independently 5 to 15; and z is 5 to 50. In another embodiment of the invention, i and k are each independently 6 to 12; and z is 15 to 35.

The nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995 or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

In an embodiment of the invention, cationic surfactants may optionally be used in the wash composition of the present invention.

One class of optional cationic surfactants includes heterocyclic ammonium salts such as cetyl or stearyl pyridinium chloride, alkyl amidoethyl pyrrylinodium methyl sulfate, and lapyrium chloride. Tetra alkyl ammonium salts are another useful class of cationic surfactants suitable for optional use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Even other useful cationic surfactants suitable for optional use include quaternized hydrolysates of silk, wheat, and keratin proteins, and it is within the scope of the invention to use mixtures of the aforementioned cationic surfactants.

If used, cationic surfactants will make up no more than 1.0% by weight of the wash composition.

When present, they typically make up from 0.01 to 0.7%, and more typically, from 0.1 to 0.5% by weight of the wash composition, including all ranges subsumed therein.

In an embodiment of this invention, the wash composition will comprise less than 0.18% polymeric quaternary ammonium compounds (including salts of the same). In another embodiment, the wash composition will comprise less than 0.1% by weight polymeric quaternary ammonium compounds. In yet another embodiment, the wash composition comprises less than 0.01% by weight polymeric quaternary ammonium compounds. In even another embodiment, the wash composition is free of polymeric quaternary ammonium compounds (i.e., 0.0%).

While not desired fatty acid soaps like sodium stearate may be included in the composition but these will make up less than 1.0% (or less than 0.5% or 0.0%) by weight of the wash composition.

Again, the wash composition of the present invention is as herein defined substantially free of sulfates and isethionates, preferably also substantially free of soaps and/or synthetic thickeners like ammonium acryloyldimethyltaurate vinylpyrrolidone copolymer, alkyl acrylate cross polymers or the like.

"Substantially free of" as used herein means each individual component making up less than 0.2% and preferably, less than 0.1%, and most preferably, less than 0.05%, and even more preferably, no (0.0%) by weight of the composition.

Wash compositions free of components defined as isethionates includes free of isethionic acid and salts thereof as well as free of surfactants classified as acyl isethionates like sodium lauroyl isethionate. Wash compositions free of components defined as sulfates includes free of components having a carbon, oxygen, sulfur bond like the surfactants sodium lauryl sulfate, sodium laureth sulfate and sodium pareth sulfate.

As to the composition surfactant amounts, the same has from 3.0 to 15%, and preferably, from 4 to 13%, and most preferably, 4.5 to 11% (or 8.5%) by weight total surfactant and the total surfactant comprises from 8 to 70%, and preferably, 10 to 65%, and most preferably,15 to 60% by weight amphoteric surfactant, zwitterionic surfactant or both based on total weight of anionic, and zwitterionic and/or amphoteric surfactant in the composition. In another embodiment, total surfactant in the wash composition is 5.85 to 7.0% and preferably, from 6.15 to 6.85% by weight based on total weight of the wash composition. In even another embodiment, the weight ratio of anionic surfactant (preferably, sodium methyl lauroyl taurate) to zwitterionic surfactant (preferably, cocoamidopropyl betaine) is from 1:1 to 1 to 2.2, and preferably, from 1:1.2 to 1 to 2.0, and most preferably, from 1:1.5 to 1.35:1.85.

Water typically makes up from 62 to 95%, and preferably, from 65 to 90%, and most preferably, from 65 to 80% by weight of the wash composition.

Adjusters suitable to modify/buffer the pH may optionally be included. Such pH adjusters include triethylamine, NaOH, KOH, H₂SO₄, HCl, C₆H₈O₇ (i.e., citric acid) or mixtures thereof. The pH adjusters are added at amounts to yield the desired final pH. The pH values may be assessed with commercial instrumentation such as a pH meter made commercially available from Thermo Scientific^{®}. Such pH values of the wash composition are typically greater than 4.25 to 8. In another embodiment of the invention, the pH of the wash composition is 4.5 to 6.5 and in another embodiment from 4.75 to 6.0 and in even another embodiment from 4.8 to 6.0.

Polyols suitable for use in the wash composition are limited only to the extent that they are usable in a wash composition for topical application. Illustrative and non-limiting examples of the polyols suitable for use in the present invention include sorbitol, glycerol, mannitol, xylitol, maltitol or mixtures thereof. In an embodiment of the invention, the polyol used is typically at least 50% by weight glycerol, based on total weight of the polyol used in the wash composition. In another embodiment of the invention, the polyol used is all glycerol (100% by weight). Polyol will typically make up from 0.0 to 25% by weight of the wash composition, and preferably, from 0.5 to 8% by weight of the wash composition, and most preferably, from 0.75 to 3.5% by weight of the wash composition.

While not required, in addition to the mixture of thickener having starch and cellulose of the present invention, it is within the scope of this invention to optionally use traditional gelling or thickening agents like ammonium acryloyldimethyltaurate vinylpyrrolidone copolymer, alkyl acrylate cross polymers or the like. If used, the acrylates include those generally classified as acrylic acid crosslinked with allyl sucrose or allyl pentaerythritol acrylic acid and C₁₀-C₃₀ alkyl acrylate crosslinked with allyl pentaerythritol. Such acrylates are sold under the Carbopol^{®} and Ultrez^{®} names and are made commercially available by Lubrizol. Other acrylates suitable for use include those which are a copolymer formed from an ester of acrylic acid and ethoxylated palm alcohol with about 25 moles of ethylene oxide and one or more monomers of acrylic acid, methacrylic acid or one of their simple esters. These acrylates are sold, for example, under the Synthalen^{®} (i.e., W200, W2000) names, and made commercially available from suppliers like 3V Sigma USA. When used such optional thickeners will make up less than 0.2%, and preferably, less than 0.1%, and most preferably, less than 0.05% by weight of the composition. Again, and even more preferably, no (0.0%) thickener in addition to starch and cellulose as described herein is used. The thickener preferred for use in the wash composition of the present invention preferably consists of the cellulose and starch as claimed. In an embodiment of the invention, cross-linked polymers and/or hydrophobically modified polymers are absent from the wash composition of the invention.

The viscosity of the wash composition (measured at 4 s⁻¹ for 20 seconds) is typically 40,000 cps or less, and preferably, from 700 to 15,000 cps, and most preferably, from 2000 to 13,500 cps.

Optional skin benefit agents suitable for use in the wash composition are limited only to the extent that they are capable of being topically applied, and suitable to remain stable in the wash composition at the desired pH.

Illustrative examples of the benefit agents suitable to include in the water portion of the wash composition are acids, like amino acids, such as arginine, valine or histidine. Additional water soluble benefit agents suitable for use include vitamin B₂, niacinamide (vitamin B₃), vitamin B5 (pantothenic acid), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water-soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water-soluble benefit agents (including mixtures) when present in the wash composition may range from 0.0 to 10%, preferably from 0.001 to 8%, and most preferably, from 0.01 to 6% (or 0.01 to 2%) by weight, based on total weight of the wash composition.

It is also within the scope of the present invention to optionally include oil soluble benefit agents. Such oil soluble benefit agents can be solubilized in the surfactants used. The only limitation with respect to such oil soluble benefit agents are that the same are suitable to provide a benefit when topically applied.

Illustrative examples of the types of oil soluble benefit agents that may optionally be used in the compositions of this invention include components like stearic acid, vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol, thiamidol (Isobutylamido thiazolyl resorcinol) or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble benefit agents suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like.

In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred.

When optional oil soluble benefit agent is used in the composition, such benefit agent typically makes up from 0.0001 to 1.5%, and preferably, from 0.001 to 0.65%, and most preferably, from 0.05 to 0.35% by weight of the wash composition. In yet another embodiment, oil soluble benefit agent makes up from 0.1 to 0.5% by weight of the total weight of the wash composition.

Other optional additives suitable for use include zinc pyrithione, octopirox and mixtures thereof. When used, they typically are employed from 0.001 to 1.5%, and preferably, from 0.01 to 1% by weight of the wash composition.

Conventional preservatives can desirably be incorporated into the wash composition to protect against the growth of potentially harmful microorganisms. Cosmetic chemists are familiar with appropriate preservatives and routinely choose them to satisfy the preservative challenge test and to provide product stability. Suitable traditional preservatives for use include hydantoin derivatives and propionate salts. Particularly preferred preservatives are iodopropynyl butyl carbamate, phenoxyethanol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin, sodium benzoate, benzyl alcohol and mixtures thereof. Other preservatives suitable for use include sodium dehydroacetate, chlorophenesin, decylene glycol, N-capryloyl glycine, N-undecylenoyl glycine and mixtures of the same. The preservatives should be selected having regard for the use of the composition and possible incompatibilities between the preservatives and other ingredients in the composition. Preservatives are preferably employed in amounts ranging from 0.01% to 2.0% by weight of the total weight of the wash composition. A preservative system that includes from 0.1 to 1.0% 1,2-octane diol based on total weight of the wash composition is suitable for use. Preservative systems substantially free of parabens and hydantoins are preferred. Sodium benzoate is an often most preferred preservative and employed typically at from 0.2 to 1.2% by weight of the wash composition.

Fragrances, fixatives, chelators (like EDTA), opacifiers (like titanium dioxide and vegetable-derived liquid dispersions like MACKADET^{®} OPR 2 made available from Solvay), inorganics and exfoliants may optionally be included in the wash composition. Each of these substances may range from about 0.03 to about 5%, preferably between 0.1 and 3%, and most preferably, from 0.1 to 2% by weight of weight of the wash composition. To the extent the exfoliants are used, those selected should be of small enough particle size so that they do not impede the performance of any packaging used to dispense the compositions of this invention.

Conventional emulsifiers having an HLB of greater than 8 may optionally be used. Illustrative examples include Tween, 40, 60, 80, polysorbate 20 and mixtures thereof. Such emulsifiers, when used for water continuous systems, make up from 0.03 to 1.5% by weight of the wash composition.

Regarding silicones like dimethicone or the like, the wash compositions will typically comprise less than 1.0% by weight silicone, more preferably, less than 0.5% by weight silicone, and most preferably, no silicone.

The wash compositions of the present invention is preferably a liquid wash composition suitable to be in an isotropic or lamellar phase.

When lamellar, the composition of the invention utilizes from 0.1 to 20%, and preferably, from 1 to 10%, and most preferably from 2 to 5% by weight of a lamellar structuring agent (based on total weight of liquid wash composition) which works in the compositions to form the lamellar phase.

Examples of fatty acids (or ester derivative, or fatty alcohol, or trihydroxystearin) which may be used as structurants are C₈-C₂₂ fatty acids like caprylic acid, lauric acid, myristic acid, oleic acid, isostearic acid, linoleic acid, linolenic acid, ricinoleic acid, elaidic acid, arachidonic acid, myristoleic acid and palmitoleic acid, mixtures thereof or the like. Ester derivatives include propylene glycol isostearate, propylene glycol oleate, glyceryl isostearate, glyceryl oleate and polyglyceryl diisostearate, or the like. The structurant is preferably a fatty acid. More preferably, the structurant is selected from the group consisting of caprylic acid, lauric acid, myristic acid or a mixture thereof.

When making wash composition of the present invention, the desired ingredients may be mixed with conventional apparatus under moderate shear and atmospheric conditions, with temperature ranging from 30 to 85°C whereby shear continues until a homogeneous product is recovered.

The packaging for the wash composition typically is not limited as long as composition can be dispensed. In an embodiment on the invention, the wash composition is sold in a pouch, bottle, jar, tube or canister. The packaging preferably allows for infinite numbers of refilling to invariably reduce plastic waste in the environment, and most preferably, has at least 50% by weight post-consumer resin. The wash compositions of the present invention may be sold in a concentrated form whereby the consumer can be instructed to add water to generate product ready for use and in an effort to reduce weight when shipping and over reliance on virgin plastic.

The Examples are provided to facilitate an understanding of the invention. They are not intended to limit the scope of the claims.

### Examples

All Samples in the Examples were prepared by mixing the ingredients with moderate shear. Temperature was varied from about 60 to 78°C and pressure while mixing was atmospheric.

The starch used in the Samples was StarDesign^{™} Care provided commercially from Cargill and the cellulose used was Vivapur^{®} COS6 provided commercially from JRS Pharma GmbH & Co.

### Example I

Base wash compositions were made with the ingredients identified below. Starch and cellulose were added to the base compositions as described in the Samples of Example II.

### WASH COMPOSITION BASE

| Ingredient | Weight Percent |
|---|---|
| Sodium methyl lauroyl taurate | 2.16 - 5.1 |
| Cocamidopropyl betaine | 0.6 - 3.8 |
| Cocamide MEA | 0.5 |
| Stearic acid | 0.3 |
| Thickener | Per Example II |
| Glycerol | 5.0 |
| Preservative | 0.4 |
| Opacifier | 0-1 |
| NaOH | To target pH |
| Citric Acid | 0.2 |
| Water | To Balance |

### Example II

The Samples (final wash compositions) of this Example II were made by including biodegradable thickener in the weight percents identified below to the base composition of Example I. Surfactant in the final compositions totaled 6.5% and pH was adjusted from 4.8 to 6.0.

### Final Wash Compositions (biodegradable thickener added)

| Sample | Starch (Weight %) | Cellulose (Weight %) | Viscosity mPa-s | Stability |
|---|---|---|---|---|
| 1 | 3.5 | 0.5 | 5,682 | Unstable |
| 2 | 3.5 | 1.0 | 8,210 | Stable |
| 3 | 3.5 | 1.5 | 10,920 | Stable |
| 4 | 4.0 | 0.5 | 5,897 | Unstable |
| 5 | 4.0 | 1.0 | 11,970 | Stable |
| 6 | 4.0 | 1.5 | 13,930 | Stable |
| 7 | 2.0 | | 270 | Unstable |
| 8 | 6.0 | | 12,310 | Unstable |

The Samples were stored at 45°C for two (2) weeks. Visual assessments of the Sample wash compositions were made by trained panelists. Unexpectedly, wash compositions made consistent with the invention (Samples 2,3,5 and 6) were stable, showing no signs of syneresis, discoloration or malodor. Moreover, the viscosity of the same remained constant over the two-week period at elevated temperature. All Samples made inconsistent with the invention (Samples 1,4,7 and 8) displayed severe separation and discoloration and were not suitable for use, even if viscosity remained stable.

After washing the hands and forearms with the compositions made consistent with the invention, all trained panelists concluded that the wash compositions rinsed off easily, left a filmless sensation and lathered well. Also, the panelists surprisingly concluded the compositions made according to the invention performed similar to those made with traditional polymeric synthetic thickeners.

## Claims

1. A wash composition comprising:
a) anionic surfactant;
b) amphoteric surfactant, zwitterionic surfactant or both;
c)
0 to 4% by weight nonionic surfactant; and
1.7 to 8.5% by weight thickener, the thickener having starch and cellulose, the starch being a hydroxypropyl starch, distarch phosphate or mixture thereof and the cellulose being a microcrystalline cellulose selected from the group consisting of carboxymethylcellulose, hydroxymethylcellulose, hydroxypropylcellulose, hydroxypropylmethylcellulose, hydroxyethylcellulose or a mixture thereof,
wherein when the thickener is present at 1.7 to less than or equal to 4.5% by weight of the composition, the weight ratio of starch:cellulose is greater than 0 and less than 4.5, and further wherein when the thickener is present at greater than 4.5% to 8.5% by weight, the weight ratio of starch:cellulose is > 0 and less than 15, and:
i) the composition has from 3.0 to 15% by weight total surfactant;
ii) the total surfactant comprises from 8 to 70% by weight amphoteric surfactant, zwitterionic surfactant or both based on total weight of anionic, and zwitterionic and/or amphoteric surfactant in the composition; and
iii) the composition has a pH from greater than 4.25 to 8,
the composition is substantially free of sulfate and isethionate, wherein substantially free means each individual component making up less than 0.2% by weight of the total composition.

2. The wash composition according to claim 1 wherein the anionic surfactant comprises a taurate and the zwitterionic surfactant is present and comprises a betaine and further wherein the composition comprises from 4 to 13% by weight total surfactant and has a pH from 4.5 to 6.5, and wherein the thickener is at least 80% by weight starch and cellulose based on total weight of the thickener in the composition.

3. The wash composition according to claims 1 and 2 wherein the pH of the composition is from 4.75 to 6 and further wherein the composition comprises from 10 to 65%, by weight amphoteric surfactant, zwitterionic surfactant or both based on total weight of anionic, and zwitterionic and/or amphoteric surfactant in the composition.

4. The wash composition according to any of the preceding claims wherein the composition comprises 12-hydroxystearic acid.

5. The wash composition according to any of the preceding claims wherein the composition is substantially free of synthetic thickener, silicone, hydantoin and paraben, wherein substantially free means each individual component making up less than 0.2% by weight of the total composition.

6. The wash composition according to claim 5 wherein the synthetic thickener is ammonium acryloyldimethyltaurate vinylpyrrolidone copolymer, alkyl acrylate crosslinked polymer or a mixture thereof.

7. The wash composition according to any of the preceding claims wherein the anionic surfactant is sodium methyl lauroyl taurate and the zwitterionic surfactant is cocamidopropyl betaine.

8. The wash composition according to any of the preceding claims wherein the anionic surfactant to zwitterionic surfactant is at a weight ratio from 1:1 to 1 to 2.2.

9. The wash composition according to any of the preceding claims wherein water makes up from 62 to 95% by weight of the wash composition.

10. The wash composition according to any of the preceding claims wherein the wash composition further comprises a water soluble or oil soluble benefit agent or both.

11. The wash composition according to any of the preceding claims wherein the wash composition further comprises 4-ethyl resorcinol, 4-hexyl resorcinol, niacinamide, thiamidol, thymol, terpineol or a mixture thereof.

12. The wash composition according to any of the preceding claims wherein the wash composition comprises no sulfates, silicones and parabens and does comprise sodium benzoate.

13. The wash composition according to any of the preceding claims wherein the wash composition further comprises zinc pyrithione, octopirox or a mixture thereof.

14. The wash composition according to any of the preceding claims wherein the anionic surfactant is taurate and the zwitterionic surfactant is a betaine.

15. The composition according to any of the preceding claims, wherein the composition is substantially free of soap, paraben and/or formaldehyde donor and comprises from 4.5 to 11% by weight total surfactant, wherein substantially free means each individual component making up less than 0.2% by weight of the total composition.

## Patentansprüche

1. Waschmittelzusammensetzung, umfassend:
a) anionisches Tensid;
b) amphoteres Tensid, zwitterionisches Tensid oder beides;
c)
0 bis 4 Gew.-% nichtionisches Tensid; und
1,7 bis 8,5 Gew.-% Verdickungsmittel, wobei das Verdickungsmittel Stärke und Cellulose aufweist, wobei die Stärke eine Hydroxypropylstärke, Distärkephosphat oder eine Mischung davon ist und die Cellulose eine mikrokristalline Cellulose ist, ausgewählt aus der Gruppe, bestehend aus Carboxymethylcellulose, Hydroxymethylcellulose, Hydroxypropylcellulose, Hydroxypropylmethylcellulose, Hydroxyethylcellulose oder einer Mischung davon,
wobei, wenn das Verdickungsmittel mit 1,7 Gew.-% bis weniger als oder gleich 4,5 Gew.-% der Zusammensetzung vorhanden ist,
das Gewichtsverhältnis von Stärke : Cellulose größer als 0 und kleiner als 4,5 ist, und
wobei ferner, wenn das Verdickungsmittel mit mehr als 4,5 bis 8,5 Gew.-% vorhanden ist, das Gewichtsverhältnis von Stärke : Cellulose größer als 0 und kleiner als 15 ist, und
i) die Zusammensetzung 3,0 bis 15 Gew.-% gesamtes Tensid enthält;
ii) das gesamte Tensid 8 bis 70 Gew.-% amphoteres Tensid, zwitterionisches Tensid oder beides, bezogen auf das Gesamtgewicht an anionischem und zwitterionischem und/oder amphoterem Tensid in der Zusammensetzung, umfasst und
iii) die Zusammensetzung einen pH-Wert von größer als 4,25 bis 8 aufweist,
die Zusammensetzung im Wesentlichen frei von Sulfat und Isethionat ist, wobei "im Wesentlichen frei" bedeutet, dass jeder einzelne Bestandteil weniger als 0,2 Gew.-% der Gesamtzusammensetzung ausmacht.

2. Waschmittelzusammensetzung nach Anspruch 1, wobei das anionische Tensid ein Taurat umfasst und das zwitterionische Tensid vorhanden ist und ein Betain umfasst und wobei die Zusammensetzung ferner 4 bis 13 Gew.-% gesamtes Tensid umfasst und einen pH-Wert von 4,5 bis 6,5 aufweist und wobei das Verdickungsmittel mindestens 80 Gew.-% Stärke und Cellulose, bezogen auf das Gesamtgewicht des Verdickungsmittels in der Zusammensetzung, umfasst.

3. Waschmittelzusammensetzung gemäß den Ansprüchen 1 und 2, wobei der pH-Wert der Zusammensetzung zwischen 4,75 und 6 liegt und wobei die Zusammensetzung ferner 10 bis 65 Gew.-% amphoteres Tensid, zwitterionisches Tensid oder beides, bezogen auf das Gesamtgewicht an anionischem und zwitterionischem und/oder amphoterem Tensid in der Zusammensetzung, umfasst.

4. Waschmittelzusammensetzung nach einem der vorhergehenden Ansprüche, wobei die Zusammensetzung 12-Hydroxystearinsäure umfasst.

5. Waschmittelzusammensetzung nach einem der vorhergehenden Ansprüche, wobei die Zusammensetzung im Wesentlichen frei von synthetischen Verdickungsmitteln, Silikon, Hydantoin und Paraben ist, wobei "im Wesentlichen frei" bedeutet, dass jeder einzelne Bestandteil weniger als 0,2 Gew.-% der gesamten Zusammensetzung ausmacht.

6. Waschmittelzusammensetzung nach Anspruch 5, wobei das synthetische Verdickungsmittel ein Ammoniumacryloyldimethyltaurat-Vinylpyrrolidon-Copolymer, Alkylacrylat-vernetztes Polymer oder eine Mischung davon ist.

7. Waschmittelzusammensetzung nach einem der vorhergehenden Ansprüche, wobei das anionische Tensid Natriummethyllauroyltaurat und das zwitterionische Tensid Cocamidopropylbetain ist.

8. Waschmittelzusammensetzung nach einem der vorhergehenden Ansprüche, wobei das anionische Tensid zum zwitterionischen Tensid in einem Gewichtsverhältnis von 1:1 bis 1:2,2 vorliegt.

9. Waschmittelzusammensetzung nach einem der vorhergehenden Ansprüche, wobei Wasser 62 bis 95 Gew.-% der Waschmittelzusammensetzung ausmacht.

10. Waschmittelzusammensetzung nach einem der vorhergehenden Ansprüche, wobei die Waschmittelzusammensetzung ferner einen wasserlöslichen oder öllöslichen Wirkstoff oder beides enthält.

11. Waschmittelzusammensetzung nach einem der vorhergehenden Ansprüche, wobei die Waschmittelzusammensetzung ferner 4-Ethylresorcin, 4-Hexylresorcin, Niacinamid, Thiamidol, Thymol, Terpineol oder eine Mischung davon enthält.

12. Waschmittelzusammensetzung nach einem der vorhergehenden Ansprüche, wobei die Waschmittelzusammensetzung keine Sulfate, Silikone und Parabene enthält, aber Natriumbenzoat enthält.

13. Waschmittelzusammensetzung nach einem der vorhergehenden Ansprüche, wobei die Waschmittelzusammensetzung ferner Zinkpyrithion, Octopirox oder eine Mischung davon enthält.

14. Waschmittelzusammensetzung nach einem der vorhergehenden Ansprüche, wobei das anionische Tensid Taurat ist und das zwitterionische Tensid ein Betain ist.

15. Zusammensetzung nach einem der vorhergehenden Ansprüche, wobei die Zusammensetzung im Wesentlichen frei von Seife, Paraben und/oder Formaldehyd-Donor ist und 4,5 bis 11 Gew.-% gesamtes Tensid umfasst, wobei "im Wesentlichen frei" bedeutet, dass jede einzelne Komponente weniger als 0,2 Gew.-% der gesamten Zusammensetzung ausmacht.

## Revendications

1. Composition de lavage comprenant :
a) un tensioactif anionique ;
b) un tensioactif amphotère, un tensioactif zwittérionique ou les deux ;
c) de 0 à 4 % en poids de tensioactif non ionique ; et
de 1,7 à 8,5 % en poids d'épaississant, l'épaississant ayant de l'amidon et de la cellulose, l'amidon étant un amidon hydroxypropylé, un phosphate de diamidon ou un mélange de ceux-ci et la cellulose étant une cellulose microcristalline choisie dans le groupe constitué par la carboxyméthylcellulose, l'hydroxyméthylcellulose, l'hydroxypropylcellulose, l'hydroxypropylméthylcellulose, l'hydroxyéthylcellulose ou un mélange de celles-ci,
dans laquelle lorsque l'épaississant est présent à raison de 1,7 à 4,5 % en poids ou moins de la composition, le rapport pondéral amidon:cellulose est supérieur à 0 et inférieur à 4,5, et en outre dans laquelle lorsque l'épaississant est présent à plus de 4,5 % à 8,5 % en poids, le rapport pondéral amidon:cellulose est > 0 et inférieur à 15, et :
i) la composition a de 3,0 à 15 % en poids de tensioactif total ;
ii) le tensioactif total comprend de 8 à 70 % en poids de tensioactif amphotère, de tensioactif zwittérionique ou des deux, en se basant sur le poids total des tensioactifs anionique et zwittérionique et/ou amphotère dans la composition ; et
iii) la composition a un pH de plus de 4,25 à 8,
la composition est sensiblement exempte de sulfate et d'iséthionate, dans laquelle sensiblement exempte signifie que chaque composant individuel représente moins de 0,2 % en poids de la composition totale.

2. Composition de lavage selon la revendication 1 dans laquelle le tensioactif anionique comprend un taurate et le tensioactif zwittérionique est présent et comprend une bétaïne et en outre dans laquelle la composition comprend de 4 à 13 % en poids de tensioactif total et a un pH de 4,5 à 6,5, et dans laquelle l'épaississant est au moins 80 % en poids d'amidon et de cellulose en se basant sur le poids total de l'épaississant dans la composition.

3. Composition de lavage selon les revendications 1 et 2 dans laquelle le pH de la composition est de 4,75 à 6 et en outre dans laquelle la composition comprend de 10 à 65 % en poids de tensioactif amphotère, de tensioactif zwittérionique ou des deux en se basant sur le poids total des tensioactifs anionique, zwittérionique et/ou amphotère dans la composition.

4. Composition de lavage selon l'une quelconque des revendications précédentes dans laquelle la composition comprend de l'acide 12-hydroxystéarique.

5. Composition de lavage selon l'une quelconque des revendications précédentes dans laquelle la composition est sensiblement exempte d'épaississant synthétique, de silicone, d'hydantoïne et de parabène, dans laquelle sensiblement exempte signifie que chaque composant individuel représente moins de 0,2 % en poids de la composition totale.

6. Composition de lavage selon la revendication 5 dans laquelle l'épaississant synthétique est un copolymère d'acryloyldiméthyltaurate d'ammonium et de vinylpyrrolidone, un polymère réticulé d'acrylate d'alkyle ou un mélange de ceux-ci.

7. Composition de lavage selon l'une quelconque des revendications précédentes dans laquelle le tensioactif anionique est le méthyllauroyltaurate de sodium et le tensioactif zwittérioniques est la cocamidopropylbétaïne.

8. Composition de lavage selon l'une quelconque des revendications précédentes dans laquelle le rapport pondéral du tensioactif anionique au tensioactif zwittérionique est de 1: 1 à 1 à 2,2.

9. Composition de lavage selon l'une quelconque des revendications précédentes dans laquelle l'eau représente de 62 à 95 % du poids de la composition de lavage.

10. Composition de lavage selon l'une quelconque des revendications précédentes dans laquelle la composition de lavage comprend en outre un agent bénéfique hydrosoluble ou liposoluble ou les deux.

11. Composition de lavage selon l'une quelconque des revendications précédentes dans laquelle la composition de lavage comprend en outre du 4-éthylrésorcinol, du 4-hexylrésorcinol, du niacinamide, du thiamidol, du thymol, du terpinéol ou un mélange de ceux-ci.

12. Composition de lavage selon l'une quelconque des revendications précédentes dans laquelle la composition de lavage ne comprend pas de sulfates, de silicones et de parabènes et comprend du benzoate de sodium.

13. Composition de lavage selon l'une quelconque des revendications précédentes dans laquelle la composition de lavage comprend en outre de la pyrithione de zinc, de l'octopirox ou un mélange de ceux-ci.

14. Composition de lavage selon l'une quelconque des revendications précédentes dans laquelle le tensioactif anionique est un taurate et le tensioactif zwittérioniques est une bétaïne.

15. Composition selon l'une quelconque des revendications précédentes dans laquelle la composition est sensiblement exempte de savon, de parabène et/ou de donneur de formaldéhyde et comprend de 4,5 à 11 % en poids de tensioactif total, dans laquelle sensiblement exempte signifie que chaque composant individuel représente moins de 0,2 % en poids de la composition totale.
